# EUROPEAN PATENT APPLICATION

(11) **EP 4 491 633 A1**
(43) Date of publication of application: **15.01.2025**
(21) Application number: 23767178.9
(22) Date of filing: 08.03.2023
(51) Int. Cl.: C07K 16/28, C07K 14/725, C07K 14/705, C12N 5/0783, A61K 35/17, A61K 39/00, A61P 35/00

(54) **PD-L1-SPECIFIC CHIMERIC ANTIGEN RECEPTOR AND IMMUNE CELL COMPRISING SAME**

(30) Priority: 08.03.2022 KR 20220029633
(71) Applicant: Vaxcell-Bio Co., Ltd., Jeollanam-do 58141 (KR)
(72) Inventor: RHEE, Joon Haeng, Gwangju 61903 (KR); LIM, Sang Bin, Daejeon 34014 (KR); PARK, Bum Chan, Daejeon 34014 (KR); PARK, Jae Eun, Daejeon 34014 (KR)
(74) Representative: Marks & Clerk LLP
(86) International application number: PCT/KR2023/003202
(87) International publication number: WO 2023/172077

(57) **Abstract**

The present invention relates to a PD-L1 specific chimeric antigen receptor and an immune cell comprising the same. Particularly, the PD-L1 specific chimeric antigen receptor comprises a binding domain that specifically binds to programmed death-ligand 1 (PD-L1), thus having excellent PD-L1 specific targeting efficiency, and exhibits excellent anticancer effects against cancer cells expressing PD-L1, and thus can be effectively used in the prevention or treatment of cancer.

## Description

### Technical Field

The present invention relates to a PD-L1 (programmed death-ligand 1) specific chimeric antigen receptor and an immune cell comprising the same. Particularly, the PD-L1 specific chimeric antigen receptor comprises a binding domain that specifically binds to PD-L1.

### Background Art

When treating solid cancer, it is difficult to completely remove cancer stem cells, remaining cancer cells, or metastatic cancer cells through surgical resection alone, so a combination treatment method that combines surgical resection with radiation therapy, chemotherapy, target therapy, and immunotherapy is being used. In particular, immunotherapy targets specific genetic characteristics of tumor cells and utilizes the body's immune system, so it has almost no side effects seen in radiation therapy or chemotherapy, and can be effective for various types of cancer.

In general, immune cells have proteins referred to as immune checkpoints on their cell membranes, which can suppress autoimmune responses. Immune checkpoints are receptors that negatively regulate the proliferation or function of T cells, which play a central role in adaptive immunity, and include receptors such as CTLA4 (cytotoxic T-lymphocyte associated protein 4), PD-1 (programmed cell death protein 1), LAG3 (lymphocyte-activation gene-3), KIR (killer cell immunoglobulin-like receptor), TIM3 (T-cell immunoglobulin and mucin-domain containing-3), VISTA (V-domain Ig suppressor of T cell activation).

Tumor cells exert immunosuppression or immune evasion through mechanisms that express ligands for these immune checkpoints. In addition, Tumor cells suppress immune function by changing the tumor microenvironment or evade immunity through T cell immune tolerance or immunoediting, etc.

As it has been revealed that cancer cells can survive from immune cells by using immune checkpoints, the development of drugs that interfere with the binding of immune checkpoint proteins and ligands on cancer cells has been actively pursued. As a result, monoclonal antibodies that can specifically bind to the PD-1/CTLA-4 proteins, immune checkpoint proteins, have been developed. The PD-1 antibody or the CTLA-4 antibody binds to the PD-1 protein and the CTLA-4 protein present on the immune cell membrane, respectively, thereby preventing these immune checkpoint proteins from binding to the ligand present on cancer cells, thereby maintaining the cancer cell killing function of the immune cells. These antibody-based immune anticancer agents include atezolizumab, avelumab, and ipilimumab.

In addition, unlike existing immunotherapy, immune cell-based immune anticancer agents have been developed that specifically bind to cancer cells and remove cancer cells through the body's immune cells. These agents include T cells (CAR T cells) that express a chimeric antigen receptor (CAR) that can directly recognize tumor-related antigens, or T cells that express an antibody sequence specific for immune checkpoint receptors on cancer cells, and are cancer therapeutic agents that enable immune cells to specifically attack cancer cells.

The structure of the currently developed chimeric antigen receptor is divided into an scFv (single chain variable fragment) portion that recognizes the antigen, a transmembrane domain, and a signaling domain that transmits signals inside a cell. Pharmaceutical companies such as Novartis, Gilead, Celgene, and Abclon, as well as clinicians, have developed CAR T cells using scFv that specifically binds to the CD 19 antigen of blood cancer cells as a treatment for blood cancer (International Publication Nos. WO201616473, WO2015157252, WO2018023025, WO2017211900, WO2017211900, and WO2016028896).

However, although CAR-based immune cell therapy that combines various genetic manipulation technologies is absolutely required for solid cancer, research results are still insufficient in the case of solid cancer due to the complex characteristics of the tumor microenvironment such as heterogeneity, which means that the tumor expresses different antigens for each patient, hypoxia, and restrictions on the movement and activity of T cells.

In addition, currently commercially available avelumab and atezolizumab bind too strongly to the antigen and have a slow dissociation rate, so they can bind to normal cells and cause serious side effects (on-target off-tumor toxicity), and thus, there are frequent reports of cases where CAR T clinical trials using the scFv of these antibodies are abandoned midway.

Accordingly, the present inventors have endeavored to develop an immune cell-based immune anticancer agent that can exhibit excellent anticancer effects not only on blood cancer but also on solid cancer. As a result, the present inventors have completed the present invention.

### Prior Art Document

### Patent Document

(Patent Document 1) Korean Patent Application Publication No. 10-2020-0119891
(Patent Document 2) Korean Patent Registration No. 10-2048477

### Detailed Description of Invention

### Technical Problem

An object of the present invention is to provide a chimeric antigen receptor comprising a binding domain that specifically binds to PD-L1.

In addition, another object of the present invention is to provide an immune cell comprising the chimeric antigen receptor.

In addition, another object of the present invention is to provide a pharmaceutical composition for preventing or treating cancer, comprising the immune cell.

### Solution to Problem

The present invention provides a chimeric antigen receptor comprising a binding domain that specifically binds to PD-L1.

The chimeric antigen receptor may further comprise at least one selected from the group consisting of a signal sequence, a hinge and transmembrane domain, and an intracellular domain.

The signal sequence may comprise a signal sequence of CD8α and may preferably comprise the amino acid sequence of SEQ ID NO: 7.

The binding domain that specifically binds to PD-L1 may be a single chain variable fragment (scFv) of an anti-PD-L1 antibody and may preferably comprise the amino acid sequence of SEQ ID NO: 8.

The hinge and transmembrane domain may comprise a hinge and transmembrane domain of CD8α and may preferably comprise the amino acid sequence of SEQ ID NO: 9.

The intracellular domain may comprise an intracellular signal region sequence selected from the group consisting of CD28, 4-1BB and CD3ζ, or a sequence selected from the group consisting of a combination thereof, and preferably, the intracellular signal region sequence of CD28 may comprise the amino acid sequence of SEQ ID NO: 10, the intracellular signal region sequence of 4-1BB may comprise the amino acid sequence of SEQ ID NO: 11, and the intracellular signal region sequence of CD3ζ may comprise the amino acid sequence of SEQ ID NO: 12.

In addition, the present invention provides a polynucleotide comprising a nucleic acid sequence encoding a chimeric antigen receptor comprising a binding domain that specifically binds to PD-L1.

In addition, the present invention provides an expression vector comprising the polynucleotide, and a virus comprising the expression vector.

In addition, the present invention provides an immune cell expressing a chimeric antigen receptor comprising a binding domain that specifically binds to PD-L1 on the surface thereof.

The immune cell may be a T cell, a MIL cell, a TIL cell, an NK cell, an NKT cell, a DC cell, or an immune cell derived from iPSC, and preferably may be a T cell.

In addition, the present invention provides a pharmaceutical composition for preventing or treating cancer, comprising an immune cell expressing a chimeric antigen receptor comprising a binding domain that specifically binds to PD-L1 on the surface thereof.

The cancer may be various types of cancer including blood cancer, and may be liver cancer, glioblastoma, gastric cancer, or large intestine cancer.

### Effects of Invention

The PD-L1 specific chimeric antigen receptor according to the present invention and an immune cell comprising the same not only have excellent PD-L1 specific targeting efficiency, but also exhibit excellent anticancer effects against cancer cells expressing PD-L1, and thus can be effectively used in the prevention or treatment of cancer.

### Brief Description of Drawings

Figure 1 shows the structure of a PD-L1 specific chimeric antigen receptor vector.
Figures 2 to 4 show the results obtained by confirming whether the introduced vector is expressed in an immune cell expressing a PD-L1 specific chimeric antigen receptor.
Figure 5 shows the results obtained by measuring the cell proliferation, viability, and CAR expression maintenance of an immune cell expressing a PD-L1 specific chimeric antigen receptor.
Figure 6 shows the results obtained by confirming the surface marker of an immune cell expressing a PD-L1 specific chimeric antigen receptor.
Figure 7 shows the results obtained by confirming whether PD-L1 is expressed in the liver cancer cell lines HepG2 and Hep3B, and the glioblastoma cell line U251.
Figure 8 shows the results obtained by confirming the cancer cell killing ability of an immune cell expressing a PD-L1 specific chimeric antigen receptor in the liver cancer cell lines HepG2 and Hep3B, and the glioblastoma cell line U251.
Figure 9 shows the results obtained by confirming the cancer cell killing ability of an immune cell expressing a PD-L1 specific chimeric antigen receptor in the gastric cancer cell line SNU638.
Figure 10 shows the results obtained by confirming the cancer cell killing ability of an immune cell expressing a PD-L1 specific chimeric antigen receptor in the gastric cancer cell line SNU638, and the large intestine cancer cell lines DLD1, LOVO and HCT15.
Figure 11 shows the results obtained by confirming the cancer cell killing ability of an immune cell expressing a PD-L1 specific chimeric antigen receptor in the gastric cancer cell line SNU638, the glioblastoma cell line U251, and the large intestine cancer cell line DLD 1.
Figure 12 shows the results obtained by confirming the cell killing ability of an immune cell expressing a PD-L1 specific chimeric antigen receptor in the gastric cancer cell line SNU638 and the normal kidney cell line HEK 293T.
Figures 13 and 14 show the results obtained by confirming the antitumor ability of an immune cell expressing a PD-L1 specific chimeric antigen receptor in the liver cancer mouse model.
Figure 15 shows the results obtained by confirming the antitumor ability of an immune cell expressing a PD-L1 specific chimeric antigen receptor in the gastric cancer mouse model.

### Best Mode for Carrying out the Invention

Hereinafter, with reference to the accompanying drawings, embodiments and examples of the present disclosure will be described in detail so that those of ordinary skill in the art to which the present invention belongs can easily practice the present invention. However, the present disclosure may be implemented in various forms and is not limited to the embodiments and examples described herein.

Throughout the present specification, when a certain part "includes" a certain component, it means that other components may be further included, rather than excluding other components, unless otherwise stated.

The present invention provides a chimeric antigen receptor (CAR) comprising a binding domain that specifically binds to PD-L1 (programmed death-ligand 1).

The binding domain that specifically binds to PD-L1 is preferably an anti-PD-L1 antibody or a fragment thereof, but is not limited thereto.

In the present invention, the "fragment" of the antibody means a fragment having an antigen binding function, and is used to mean scFv, Fab, F(ab')2, and a Fv fragment, etc.

The fragment of the anti-PD-L1 antibody included in the PD-L1 specific chimeric antigen receptor of the present invention may be in the form of scFv, and may comprise the amino acid sequence of SEQ ID NO: 8, and may have at least 70%, preferably at least 80%, more preferably at least 90%, 95%, 97%, or 99% sequence identity to the amino acid sequence of SEQ ID NO: 8.

In the present invention, "programmed death-ligand 1 (PD-L1)" is a ligand for "programmed death receptor 1 (PD-1)", an immunosuppressive receptor mainly expressed on activated T and B cells. When PD-1 and the ligand PD-L1 or PD-L2 bind, antigen receptor signaling can be negatively regulated. The ligand for PD-1 can be constitutively expressed or induced by a number of cell types including non-hematopoietic tissues and various tumor types. PD-L1 is weakly expressed on B cells, T cells, myeloid cells, and dendritic cells (DCs), but is also expressed on peripheral cells, pseudo-microvascular endothelial cells, and non-lymphoid organs such as the heart and lungs. However, overexpression in tumor cells is mainly induced by interferon gamma in the tumor microenvironment, or expression is induced in immune cells existing in the tumor microenvironment by the immune evasion mechanism of tumor cells. In contrast, PD-L2 is found only on macrophages and dendritic cells. The expression pattern of PD-1 ligands suggests a role for PD-1 in maintaining peripheral tolerance and can contribute to modulating auto-reactive T-cell and B-cell responses in the periphery.

In the present invention, "chimeric antigen receptor (CAR)" refers to an extracellular antigen-binding domain fused to an intracellular signaling domain. CARs can be expressed on T cells or NK cells to increase cytotoxicity. In general, the extracellular antigen-binding domain is a single chain variable fragment (scFv) specific for an antigen found on a cell of interest. Based on the specificity of the scFv domain, cells expressing a specific antigen on the cell surface are targeted. The scFv domain can be engineered to recognize any antigen, including tumor-specific antigens and virus-specific antigens. For example, PD-L1 CAR recognizes PD-L1, a cell surface marker expressed by some cancers.

The PD-L1 specific chimeric antigen receptor of the present invention may further comprise at least one selected from the group consisting of a signal sequence (signal peptide; SP), a hinge and transmembrane domain (transmembrane domain; TM), and an intracellular domain, together with a binding domain that specifically binds to PD-L1.

The signal sequence included in the PD-L1 specific chimeric antigen receptor of the present invention is preferably a signal sequence of CD8α, but is not limited thereto, and may preferably comprise the amino acid sequence of SEQ ID NO: 7, and may have at least 70%, preferably at least 80%, more preferably at least 90%, 95%, 97%, or 99% sequence identity to the amino acid sequence of SEQ ID NO: 7.

The signal sequence according to the present invention and the binding domain that specifically binds to PD-L1 constitute an extracellular domain of the chimeric antigen receptor. The extracellular domain is a site where the main signal is transmitted, exists outside the cell membrane, and is a domain for specifically recognizing PD-L1.

The hinge and transmembrane domain included in the PD-L1 specific chimeric antigen receptor of the present invention is preferably a hinge and transmembrane domain derived from CD8α, but is not limited thereto, and may preferably comprise the amino acid sequence of SEQ ID NO: 9, and may have at least 70%, preferably at least 80%, more preferably at least 90%, 95%, 97%, or 99% sequence identity to the amino acid sequence of SEQ ID NO: 9.

The intracellular signaling domain according to the present invention is a part located inside the cell membrane of a T cell, i.e., in the cytoplasm, and refers to a site that activates an immune response of an immune cell when an antibody bound to an extracellular domain binds to a target antigen.

The intracellular domain of the PD-L1 specific chimeric antigen receptor of the present invention may comprise two or more intracellular signaling domains. The intracellular signaling domain is preferably a CD28-derived intracellular signaling domain or a 4-1BB-derived intracellular signaling domain linked to a CD3ζ-derived intracellular signaling domain, but is not limited thereto, and preferably, the CD28-derived intracellular signaling domain may comprise the amino acid sequence of SEQ ID NO: 10, the 4-1BB-derived intracellular signaling domain may comprise the amino acid sequence of SEQ ID NO: 11, and the CD3ζ-derived intracellular signaling domain may comprise the amino acid sequence of SEQ ID NO: 12, and may have at least 70%, preferably at least 80%, more preferably at least 90%, 95%, 97%, or 99% sequence identity to the amino acid sequence of SEQ ID NO: 10, SEQ ID NO: 11, or SEQ ID NO: 12.

In addition, the present invention provides an immune cell expressing a chimeric antigen receptor comprising a binding domain that specifically binds to PD-L1.

The immune cell may be a T cell, a MIL cell, a TIL cell, an NK cell, an NKT cell, a DC cell, or an immune cell derived from iPSC, but is not limited thereto, and preferably may be a T cell.

According to one embodiment of the present invention, the immune cell may be described as an "anti-PD-L1 CAR T cell" and designated as "VYPC1" or "VYPC2".

The immune cell of the present invention may exhibit a killing ability against a cancer (tumor) cell expressing PD-L1. According to one embodiment, the immune cell of the present invention (for example, a T cell) may exhibit a killing ability against a liver cancer cell or a glioblastoma cell. For example, the liver cancer cell or glioblastoma cell may express PD-L1.

In addition, the present invention provides a pharmaceutical composition for preventing or treating cancer, comprising an immune cell comprising a chimeric antigen receptor comprising a binding domain that specifically binds to PD-L1. More specifically, the present invention provides a pharmaceutical composition for preventing or treating malignization and metastasis of a tumor.

A pharmaceutical composition comprising an immune cell expressing the chimeric antigen receptor according to the present invention may further comprise a pharmaceutically acceptable excipient.

As used herein, the term " cancer (tumor)" refers to or describes a physiological condition of a mammal that is typically characterized by unregulated cell growth and proliferation. The cancer (tumor) to which the composition is applied typically includes cancers that respond to immunotherapy, and cancers that have not been related to immunotherapy to date. In the present invention, the cancer (tumor) is preferably a PD-L1-positive cancer, and may be a liver cancer cell, a glioblastoma cell, a gastric cancer cell, or a large intestine cancer cell. In addition, the present invention includes refractory or relapsed cancers whose growth can be inhibited using the antibody of the present invention or a fragment thereof.

In addition, the present invention provides a nucleic acid sequence encoding the chimeric antigen receptor according to the present invention as described above. The polynucleotide (nucleic acid) encoding the antigen receptor of the present invention can be modified by codon optimization, which is due to the degeneracy of codons, and it will be well understood by those of ordinary skill in the art that there are many nucleotide sequences encoding the polypeptide or a variant fragment thereof. Some of these polynucleotides (nucleic acids) have minimal homology to the nucleotide sequence of any naturally occurring gene.

In addition, the present invention provides a vector comprising the nucleic acid sequence according to the present invention and a virus comprising the vector according to the present invention.

As used herein, the term "prevention" refers to any action of inhibiting or delaying the onset of a disease by administration of a composition, and "treatment" refers to any action in which symptoms of a subject suspected of and suffering from a disease are improved or beneficially changed by administration of a composition.

Hereinafter, the present invention will be described in more detail through the examples, but the following examples are for illustrative purposes only and are not intended to limit the scope of the present invention.

### [Example 1]

### Production of recombinant lentiviral vector

The following strains and vectors were prepared for the production of a recombinant plasmid vector expressing a chimeric antigen receptor (Anti-PD-L1 CAR) comprising an scFv that specifically binds to PD-L1.

### 1-1. Preparation of vector

A phagemid DNA comprising a nucleic acid sequence (SEQ ID NO: 2) of an scFv that specifically binds to human PD-L1 was provided by the research team at Y-Biologics. The sequence information and production method of phagemid DNA comprising the nucleic acid sequence of the scFv are described in detail in Korean Patent Registration No. 10-1069146, and the phagemid DNA sequence information of the VH and VL chains used as scFv below are also the same. Specifically, the basic framework of the expression vector was the pLenti7.3/V5-DEST vector from Invitrogen, and the nucleic acid sequences expressing two types of chimeric antigen receptors consisting of [CD8α signal sequence - anti-PD-L1 scFv - CD8α H&TM - CD28 or 4-1BB signaling domain - CD3ζ signaling domain] were included between the 5' UTR and 3' UTR of the pLenti7.3/V5-DEST vector (pLenti7.3::CD8α signal sequence-anti-PD-L1 scFv-CD8 H&T-CD28-CD3ζ vector, VYPC1; pLenti7.3::CD8α signal sequence-anti-PD-L1 scFv-CD8 H&T-4-1BB-CD3ζ vector, VYPC2).

The nucleic acid sequences constituting the PD-L1 specific chimeric antigen receptor in the recombinant plasmid vector are shown in Table 1 below.

**[Table 1]**

| SEQ ID NO | Sequence name | Sequence |
|---|---|---|
| SEQ ID NO: 1 | CD8α signal sequence nucleotide | ATGGCCCTGCCTGTGACAGCTTTGCTGCC TCTGGCTCTGCTGCTGCATGCTGCTAGACCT |
| SEQ ID NO: 2 | PD-L1 scFv nucleotide | CAGATGCAGCTGGTGCAGTCTGGGGCTGAGG TGAAGAAGCCTGGGTCCTCGGTGAAGGTCTC CTGCAAGGCTTCTGGAGGCACCTTCAGCAGC TATGCTATCAGCTGGGTGCGACAGGCCCCTGG ACAAGGGCTTGAGTGGATGGGAAGGATCATC CCTATCCTTGGTATAGCAAACTACGCACAGAA GTTCCAGGGCAGAGTCACGATTACCGCGGAC AAATCCACGAGCACAGCCTACATGGAGCTGA GCAGCCTGAGATCTGAGGACACGGCCGTGTA TTACTGTGCGAAACCGAGAGATGGCTACAATT TGGTTGCTTTTGATATCTGGGGCCAAGGGACG ATGGTCACCGTCTCCTCAGGCCTCGGGGGCCT CGGAGGAGGAGGTAGTGGCGGAGGAGGCTC CGGTGGATCCAGCGGTGTGGGTTCCCAGCTC GTGCTGACTCAGCCGCCCTCAGTGTCTGGGG CCCCAGGGCAGAGGGTCACCATCTCCTGCAC TGGGAGCAGCTCCAACATCGGGGCAGGTTAT GATGTACACTGGTATCAGCAACTTCCAGGAGC AGCCCCCAAACTCCTCATCTATGGCGACATCA ATCGGCCCTCAGGGGTCCCTGACCGATTCTCT GGCTCCAAGTCTGGCATCTCAGCCTCCCTGGC TATCACTGGGCTCCAGGCTGAGGACGAGGCT GATTATTACTGCCAGTCCTATGACAGCAGCCT |
| | | GAGTGGGGGGGTGTTCGGCGGAGGGACCAA GCTGACCGTCCTA |
| SEQ ID NO: 3 | CD8α H&TM nucleotide | ACCACCACACCAGCTCCACGGCCTCCAACAC CAGCACCTACAATTGCCTCTCAGCCCCTGTCT CTGAGGCCAGAAGCTTGTAGACCTGCTGCAG GCGGAGCCGTGCATACCAGAGGACTGGATTT CGCCTGCGACATCTACATTTGGGCCCCTCTGG CTGGAACATGTGGCGTCCTGCTGCTGTCTCTC GTGATCACCCTGTACTGC |
| SEQ ID NO: 4 | CD28 nucleotide | AGAAGCAAGAGAAGCCGGCTGCTGCACAGC GACTACATGAACATGACCCCTAGACGGCCCG GACCTACCAGAAAGCACTACCAGCCTTACGC TCCTCCTCGGGACTTTGCCGCCTATAGATCC |
| SEQ ID NO: 5 | 4-1BB nucleotide | AAGCGGGGCAGAAAGAAGCTGCTGTACATCT TCAAGCAGCCCTTCATGCGGCCCGTGCAGAC CACACAAGAGGAAGATGGCTGCTCCTGCAGA TTCCCCGAGGAAGAAGAAGGCGGCTGCGAGC TG |
| SEQ ID NO: 6 | CD3ζ nucleotide | AGAGTGAAGTTCTCTAGATCTGCCGACGCTCC TGCCTACCAGCAGGGACAGAATCAGCTGTAC AACGAGCTGAATCTGGGGCGCAGAGAAGAGT ACGACGTGCTGGATAAGAGAAGAGGCAGAGA TCCCGAGATGGGCGGCAAGCCCCAGAGAAGA AAGAATCCCCAAGAGGGCCTGTATAATGAGCT GCAGAAAGACAAGATGGCCGAGGCCTACAGC GAGATCGGAATGAAGGGCGAACGCAGAAGA GGAAAGGGCCACGACGGACTGTATCAGGGCC TGAGCACAGCCACCAAGGATACCTATGATGCC CTGCACATGCAGGCCCTGCCACCTAGG |

The nucleic acid sequence of Table 1 above can be expressed in immune cells, and a PD-L1 specific chimeric antigen receptor consisting of the amino acid sequence of Table 2 below can be expressed on the cell surface.

**[Table 2]**

| SEQ ID NO | Sequence name | Sequence |
|---|---|---|
| SEQ ID NO: 7 | CD8α signal sequence amino acid | MALPVTALLLPLALLLHAARP |
| SEQ ID NO: 8 | PD-L1 scFv amino acid | QMQLVQSGAEVKKPGSSVKVSCKASGGTFSSY AISWVRQAPGQGLEWMGRIIPILGIANYAQKFQ GRVTITADKSTSTAYMELSSLRSEDTAVYYCAKP RDGYNLVAFDIWGQGTMVTVSSGLGGLGGGGS GGGGSGGSSGVGSQLVLTQPPSVSGAPGQRVTI SCTGSSSNIGAGYDVHWYQQLPGAAPKLLIYG DINRPSGVPDRFSGSKSGISASLAITGLQAEDEA DYYCQSYDSSLSGGVFGGGTKLTVL |
| SEQ ID NO: 9 | CD8α H&TM amino acid | TTTPAPRPPTPAPTIASQPLSLRPEACRPAAGGAV HTRGLDFACDIYIWAPLAGTCGVLLLSLVITLYC |
| SEQ ID NO: 10 | CD28 amino acid | RSKRSRLLHSDYMNMTPRRPGPTRKHYQPYAP PRDFAAYRS |
| SEQ ID NO: 11 | 4-1BB amino acide | KRGRKKLLYIFKQPFMRPVQTTQEEDGCSCRFP EEEEGGCEL |
| SEQ ID NO: 12 | CD3ζ amino acid | RVKFSRSADAPAYQQGQNQLYNELNLGRREEY DVLDKRRGRDPEMGGKPQRRKNPQEGLYNEL QKDKMAEAYSEIGMKGERRRGKGHDGLYQGL STATKDTYDALHMQALPPR |

### 1-2. Preparation of cells for replication of vector

The host cells for *E. coli* Stbl3 (F- *mcrB mrrhsd*S20(rB-, mB-) *recA*13 *sup*E44 *ara-*14 *gal*K2 *lac*Y1 *pro*A2 *rps*L20(Str^{R}) *xyl*-5 λ *-leumtl*-1) (Invitrogen^{™}) were cultured using LB (Luria-Bertani) solid medium (Difco Laboratories, USA) under conditions of 37 °C and 200 rpm, and 100 µg/ml ampicillin was added to all media to prepare the host cells. Thereafter, a single colony of each cultured host cell was inoculated into LB liquid medium (Difco Laboratories, USA) and cultured, and then inoculated into LB liquid medium at a concentration of 1% and cultured at 37 °C to O.D. 0.4 to 0.6, and then washed with CaCl₂ buffer to prepare the host cells.

### 1-3. Production of transformed cells for mass production of vector

Each of the vectors prepared above was mixed with the host cells, and then the mixture was left on ice for 10 minutes and treated at 42 °C for 90 seconds and on ice for 3 minutes to produce transformed cells into which each vector was introduced. The produced transformed cells were plated on the LB solid medium containing antibiotics and then cultured at 37 °C. Through this, each vector was prepared in large quantities.

### [Example 2]

### Production of recombinant lentiviral vector expressing PD-L1 specific chimeric antigen receptor

The lentiviral vector of Example 1 above (a lentiviral vector excluding the MSLN scFv sequence from the pLenti7.3::CD8α signal sequence-MSLN scFv-CD8 H&T-CD28-CD3ζ vector) was produced. First, inverse PCR was performed using the vector as a template, the CPL-F primer, the CPL-R primer, and the CloneAmp HiFi PCR premix (Takara) shown in Table 3 below. The amplified vector was isolated and purified to prepare an MSLN scFv-free chimeric antigen receptor vector.

The PD-L1 scFv sequence was prepared by amplifying the phagemid DNA sequence provided by Y-Biologics by PCR using the VYPC-F primer and VYPC-R primer shown in Table 3 below, followed by isolation and purification.

**[Table 3]**

| SEQ ID NO | Sequence name | Sequence |
|---|---|---|
| SEQ ID NO: 13 | CPL F | ACCACCACACCAGCTCC |
| SEQ ID NO: 14 | CPL R | AGGTCTAGCAGCATGCAG |
| SEQ ID NO: 15 | VYPC F | CTGCATGCTGCTAGACCTCAGATGCAGCTGGT GCAG |
| SEQ ID NO: 16 | VYPC R | TGGAGCTGGTGTGGTGGTTAGGACGGTCAGC TTGGTCC |

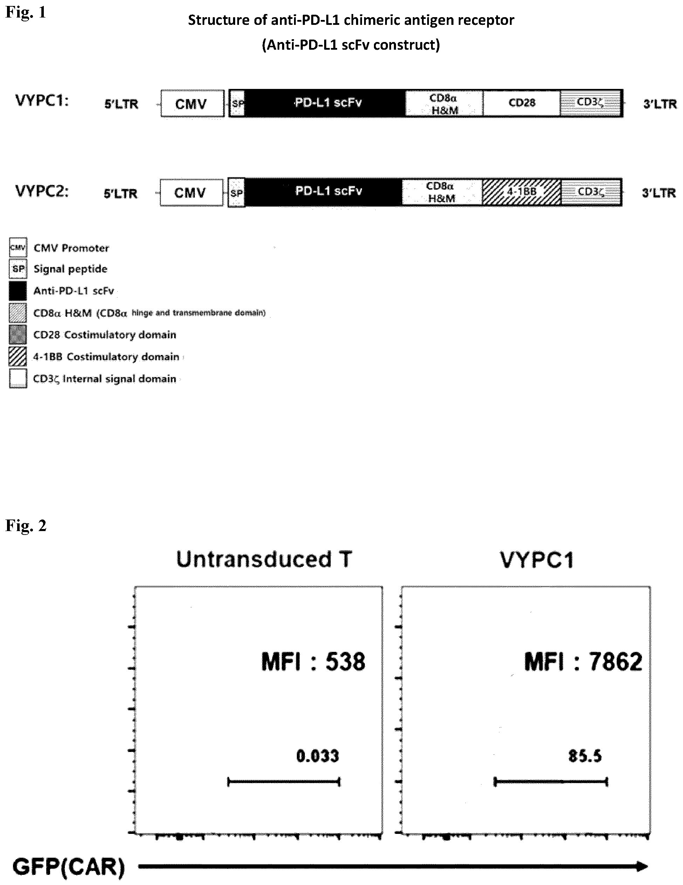

The above vector and PD-L1 scFv sequence were inserted into the site where the MSLN scFv sequence was present through homologous recombination method using the Overlap cloner DNA cloning Kit (Elpsibio), thereby producing a recombinant lentiviral vector (pLenti7.3-anti-PD-L1 chimeric antigen receptor vector) expressing a chimeric antigen receptor comprising PD-L1 scFv. The produced PD-L1 specific chimeric antigen receptor vectors (recombinant lentiviral vectors for anti-PD-L1 CAR T) were designated as pLenti7.3::VYPC1 and pLenti7.3::VYPC2, and their structures are shown in Figure 1.

### [Example 3]

### Production of immune cells (VYPC1 and VYPC2) expressing PD-L1 specific chimeric antigen receptor

### 3-1. Production of lentivirus comprising recombinant lentiviral vector

A lentivirus comprising the PD-L1 specific chimeric antigen receptor vector (pLenti7.3::VYPC1 and pLenti7.3::VYPC2) produced in Example 2 above was prepared.

### 3-2. Preparation of immune cells and transduction via virus infection

Peripheral blood mononuclear cells (PBMCs) were isolated from human donor blood using Lymphoprep^{™} (STEMCELL). Thereafter, human CD3+ T cells were isolated from the isolated PBMCs by a positive selection method using CD3 microbeads (Miltenyi Biotech).

The anti-CD3/anti-CD28 magnetic beads (anti-CD3/CD28 Dynabead; Gibco) were mixed with the isolated human CD3+ T cells at a ratio of 1:1. After 24 hours of mixing, the activated magnetic beads were removed using a MACSiMAG separator device, and the cells were washed using RPMI-1640 to activate the CD3+ T cells.

T cells infected with lentivirus were produced by a virus infection method using Polybrene (Sigma Aldrich). Specifically, the activated human CD3+ T cells were infected with a lentivirus comprising the PD-L1 specific chimeric antigen receptor vector. After 24 hours, the virus medium was replaced. Cell proliferation began 48 hours after infection, and the produced immune cells (anti-PD-L1 CAR T cells) were designated as VYPC1 and VYPC2.

### [Example 4]

### Evaluation 1 of immune cells (VYPC1 and VYPC2) expressing PD-L1 specific chimeric antigen receptor

### 4-1. Transduction rate of anti-PD-L1 CAR T cells (VYPC1)

Whether the PD-L1 specific chimeric antigen receptor vector introduced into the anti-PD-L1 CAR T cells (VYPC1) produced in Example 3 above was properly expressed were confirmed using FACS (fluorescence-activated cell sorting).

The Attune NxT flow cytometer (Thermo Fisher Scientific) was used for the experiment. For each immune cell, whether the introduced vector was expressed was confirmed using an anti-GFP (Green Fluorescent Protein) antibody. Since the GFP expression site is present in the vector, the analysis results using an anti-GFP antibody are measured high in immune cells that express this vector in large quantities.

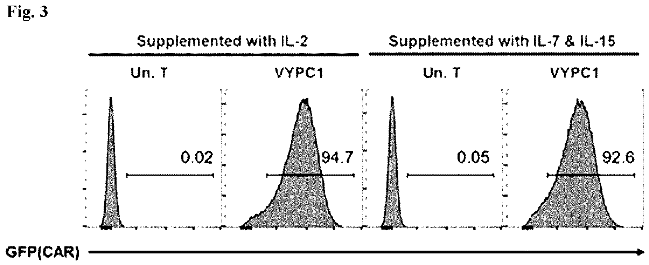

As shown in Figure 2, as a result of comparative analysis of the expression rate of the introduced vector (PD-L1 specific chimeric antigen receptor sequence) by FACS, the anti-PD-L1 CAR T cells (VYPC1) showed an expression rate of about 85.5% under general IL-2 culture conditions. In addition, as shown in Figure 3, as a result of analysis of the transduction rate after treatment with cytokines IL-2, IL-7, and IL-15 by FACS, the anti-PD-L1 CAR T cells (VYPC1) all showed 90% or more.

Therefore, it was confirmed that the PD-L1 specific chimeric antigen receptor was properly expressed in the produced anti-PD-L1 CAR T cells (VYPC1).

### 4-2. Transduction rate of anti-PD-L1 CAR T cells (VYPC1 and VYPC2) according to cytokine

In the same manner as in Example 3-2 above, peripheral blood mononuclear cells (PBMCs) were isolated from human donor blood using Lymphoprep^{™} (STEMCELL). Thereafter, human CD3+ T cells were isolated from the isolated PBMCs by a positive selection method using CD3 microbeads (Miltenyi Biotech).

The anti-CD3/anti-CD28 magnetic beads (anti-CD3/CD28 Dynabead; Gibco) were mixed with the isolated human CD3+ T cells at a ratio of 1:1. After 24 hours of mixing, the activated magnetic beads were removed using a MACSiMAG separator device, and the cells were washed using RPMI-1640 to activate the CD3+ T cells, and the cells were divided into three groups (Group 1, cultured with IL-2; Group 2, cultured with IL-7 and IL-15; Group 3, cultured with IL-2, IL-7, and IL-15) and cultured.

T cells infected with lentivirus were produced by a virus infection method using Polybrene (Sigma Aldrich). Specifically, the activated human CD3+ T cells were infected with a lentivirus comprising the PD-L1 specific chimeric antigen receptor vector. After 24 hours, the virus medium was divided into three groups and replaced. On the 6th day, whether the introduced vector was expressed was confirmed for each immune cell using an anti-GFP (Green Fluorescent Protein) antibody using the Attune NxT flow cytometer (Thermo Fisher Scientific).

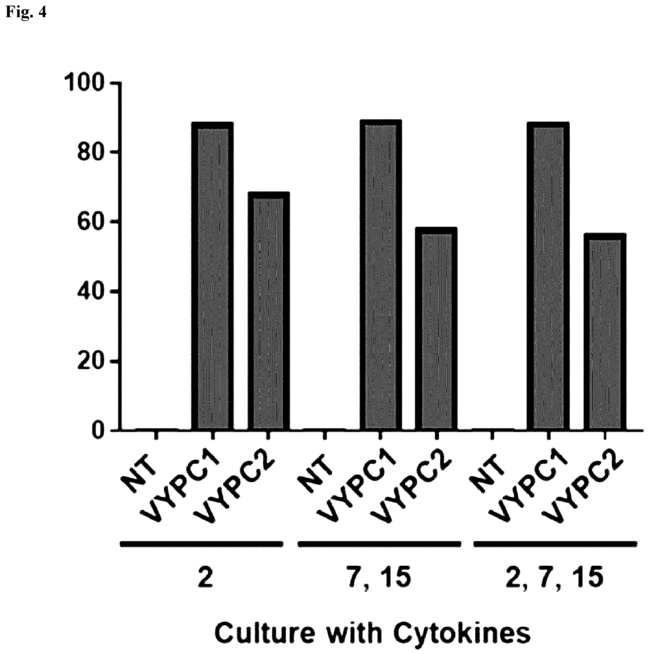

As a result, as shown in Figure 4, both VYPC1 and VYPC2 showed high expression rates, and in particular, VYPC1 consistently showed a high CAR transduction rate of 80% or more in all cytokine cultures.

Therefore, it was confirmed that the PD-L1 specific chimeric antigen receptor was properly expressed in the produced anti-PD-L1 CAR T cells (VYPC1 and VYPC2).

### [Example 5]

### Evaluation 2 of immune cells (VYPC1) expressing PD-L1 specific chimeric antigen receptor

In order to measure the cell proliferation, viability, and CAR expression maintenance of the anti-PD-L1 CAR T cell (VYPC1) produced in Example 3 above, the following was performed.

Human peripheral blood T cells were transduced with lentivirus comprising an anti-PD-L1 scFv CAR the next day after activation with CD3 and CD28 magnetic beads, and then the total cell count and viable cell count were measured every two days using NucleoCounter^{®} NC-250^{™} automated cell counter (ChemoMetec), and the number of GFP positive cells was measured using the Attune FACS equipment.

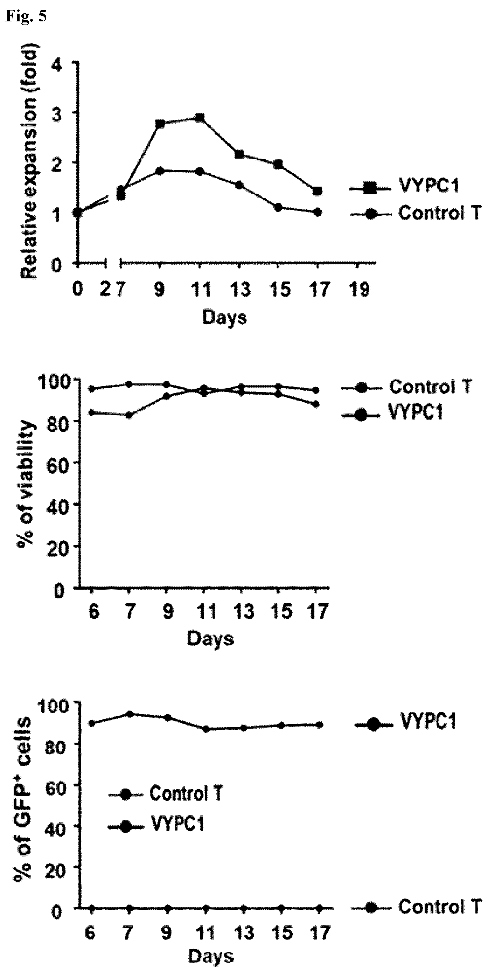

As a result, as shown in Figure 5, the anti-PD-L1 CAR T cells (VYPC1) showed an more excellent cell proliferation rate compared to that of the control group, and the viability was similar to that of the control group. In addition, it was shown to maintain a high level of chimeric antigen receptor (CAR) expression of 80% or more.

Therefore, it was confirmed that the produced anti-PD-L1 CAR T cells (VYPC1) maintained excellent cell proliferation and expression of chimeric antigen receptors.

### [Example 6]

### Evaluation 3 of immune cells (VYPC1) expressing PD-L1 specific chimeric antigen receptor

In order to confirm the surface marker of the anti-PD-L1 CAR T cells (VYPC1) produced in Example 3 above, the following was performed.

Activation of T cells was confirmed using anti-CD25-PE antibody (PE Mouse Anti-Human CD25, BD Pharmingen^{™}) and anti-CD69-APC antibody (APC Mouse Anti-Human CD69, BD Pharmingen^{™}), and inhibition was predicted using the immune cell inhibitory factors anti-LAG-3-PE antibody (Thermofisher), anti-PD-1-PE-Cy7 antibody (Thermofisher), anti-CTL-4-APC antibody (Thermofisher), TIGIT-APC-Cy7 antibody (Thermofisher), and anti-TIM-3-BV421 (Thermofisher) antibody. Since the GFP expression site is present in the vector, the analysis results using an anti-GFP antibody are measured high in immune cells that express this vector in large quantities.

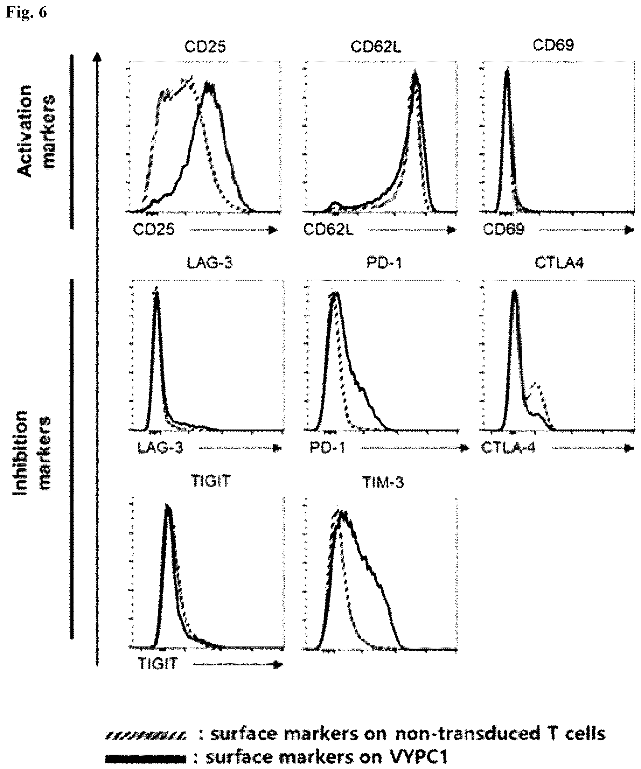

As a result, as shown in Figure 6, it was shown that in the case of the anti-PD-L1 CAR T cells (VYPC1), CD25, a cell growth activation marker, was expressed higher than in the control group, and CD69, an activation marker of cytotoxic T cells, was increased to the level similar to that of the control group. Meanwhile, it was shown that the immune cell inhibitory factors LAG-3 and TIGIT were at low levels similar to those of the control group. In addition, it was shown that the inhibitory factors PD-1, CTLA4, and TIM-3 were at levels similar to or slightly higher than those of the control group, but did not significantly affect the cancer killing efficacy.

Therefore, it was confirmed that the produced anti-PD-L1 CAR T cells (VYPC1) were activated.

### [Example 7]

### Evaluation of in vitro cancer cell killing ability of immune cells (VYPC1) expressing PD-L1 specific chimeric antigen receptor against liver cancer and glioblastoma

In order to evaluate the anticancer ability of the anti-PD-L1 CAR T cells (VYPC1) produced in Example 3 above against the PD-L1 specific antigen, the liver cancer cell lines HepG2 and Hep3B and the glioblastoma cell line U251, which express PD-L1, were each co-cultured with the anti-PD-L1 CAR T cells (VYPC1) to measure the cancer cell killing ability.

### 7-1. Evaluation of PD-L1 expression in cancer cell lines

The liver cancer cell lines HepG2 and Hep3B; and the glioblastoma cell line U251 were purchased and used from the ATCC (American Type Culture Collection). In order to confirm whether PD-L1 is expressed in these cancer cell lines and whether PD-L1 is expressed dependently on IFNγ secreted as the immune response from immune cells becomes stronger, whether the PD-L1 antigen was expressed and the level of PD-L1 antigen expression were analyzed by FACS using an anti-PD-L1 antibody.

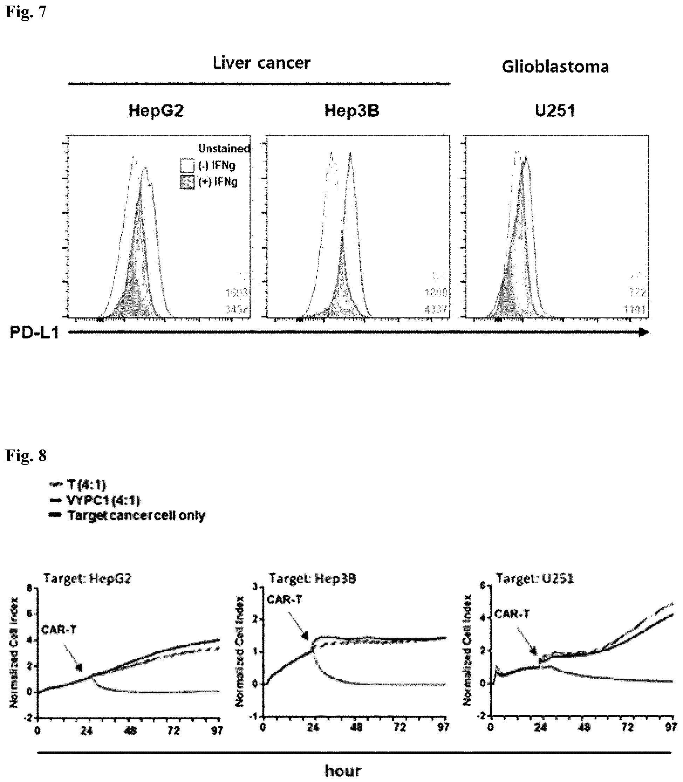

As shown in Figure 7, it was confirmed that PD-L1 was more expressed by IFNγ in both the liver cancer cell lines HepG2 and Hep3B; and the glioblastoma cell line U251. Using this, the cancer cell killing ability of the anti-PD-L1 CAR-T (VYPC1) cells targeting PD-L1 can be confirmed.

### 7-2. Evaluation of cancer cell killing ability of anti-PD-L1 CAR T cells (VYPC1)

The cancer cell lines in which expression of the PD-L1 antigen was confirmed in Example 5-1 above were seeded in a 96-well plate at 1×10⁴ cells per well and placed in BSC for 30 minutes, and then the cancer cells were cultured in a cell incubator under conditions of 37 °C and 5% CO₂ for 24 hours. Thereafter, the cancer cells were treated with 4×10⁴ anti-PD-L1 CAR T cells (VYPC1) per well and co-cultured for 72 to 90 hours, and the survival and cell movement activity of the cells were analyzed by RTCA (xCELLigence, ACEA Biosciences, Inc.). RTCA measures the resistance value called impedance that occurs during cell growth, and various cell responses (changes in cell number, proliferation rate, cell size, cell shape, and substrate attachment quality) can be automatically measured in a kinetic manner. The resistance value detected by the cell is expressed as an indicator called Cell Index (CI), and changes in the CI value indicate changes in cell shape.

As shown in Figure 8, as a result of comparing the killing ability of transduced anti-PD-L1 CAR T cells (VYPC1) and untransduced human T cells (control group) against cancer cells, it was confirmed that when treated with VYPC1 of the present invention, the cancer cells were completely killed in both the liver cancer cell lines HepG2 and Hep3B; and the glioblastoma cell line U251.

Therefore, it can be seen that the anti-PD-L1 CAR T cells (VYPC1) according to the present invention have excellent cancer cell killing ability against malignant tumor cells including liver cancer and glioblastoma expressing PD-L1.

### [Example 8]

### Evaluation of in vitro cancer cell killing ability of immune cells (VYPC1) expressing PD-L1 specific chimeric antigen receptor against gastric cancer

In order to evaluate the anticancer ability of the anti-PD-L1 CAR T cells (VYPC1) produced in Example 3 above against the PD-L1 specific antigen, the gastric cancer cell line SNU638 expressing PD-L1 was co-cultured with the anti-PD-L1 CAR T cells (VYPC1) to measure the cancer cell killing ability.

The gastric cancer cell line SNU638 in which expression of the PD-L1 antigen was confirmed was seeded in a 96-well plate at 1×10⁴ cells per well and placed in BSC for 30 minutes, and then the cancer cells were cultured in a cell incubator under conditions of 37 °C and 5% CO₂ for 24 hours. Thereafter, the cancer cells were treated with 0.5×10⁴ (0.5:1) to 2×10⁴ (2:1) anti-PD-L1 CAR T cells (VYPC1) per well and co-cultured for 72 to 90 hours, and the survival and cell movement activity of the cells were analyzed by RTCA (xCELLigence, ACEA Biosciences, Inc.). At this time, the culture was performed by adding different cytokines (IL-2 and IL-7+IL-15) to the medium used. RTCA measures the resistance value called impedance that occurs during cell growth, and various cell responses (changes in cell number, proliferation rate, cell size, cell shape, and substrate attachment quality) can be automatically measured in a kinetic manner. The resistance value detected by the cell is expressed as an indicator called Cell Index (CI), and changes in the CI value indicate changes in cell shape.

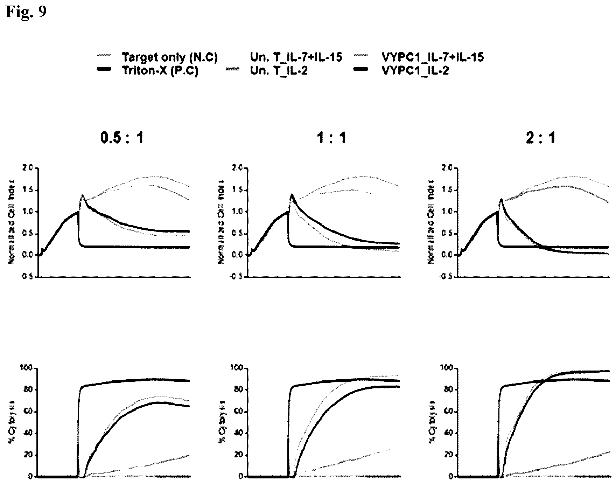

As shown in Figure 9, as a result of comparing the killing ability of transduced anti-PD-L1 CAR T cells (VYPC1) and untransduced human T cells (control group) against cancer cells, it was confirmed that when treated with VYPC1 of the present invention, the cancer cells were killed. In addition, it was confirmed that the cancer cells were killed faster when the ratio of VYPC1 to cancer cells increased, and that the cancer cells were killed faster when cultured with IL-7 and IL-15 than when cultured with IL-2.

Therefore, it can be seen that the anti-PD-L1 CAR T cells (VYPC1) according to the present invention have excellent cancer cell killing ability against malignant tumor cells including gastric cancer expressing PD-L1.

### [Example 9]

### Evaluation of cancer cell killing ability of immune cells (VYPC1) expressing PD-L1 specific chimeric antigen receptor against gastric cancer and large intestine cancer

In order to evaluate the anticancer ability of the anti-PD-L1 CAR T cells (VYPC1) produced in Example 3 above against the PD-L1 specific antigen, the gastric cancer cell line SNU638 and the large intestine cancer cell lines DLD1, LOVO and HCT15, which express PD-L1, were each co-cultured with the anti-PD-L1 CAR T cells (VYPC1) to measure the cancer cell killing ability.

The cancer cell lines in which expression of the PD-L1 antigen was confirmed were seeded in a 96-well plate at 1×10⁴ cells per well and placed in BSC for 30 minutes, and then the cancer cells were cultured in a cell incubator under conditions of 37 °C and 5% CO₂ for 24 hours. Thereafter, the cancer cells were treated with 4×10⁴ anti-PD-L1 CAR T cells (VYPC1) per well and co-cultured for 72 to 90 hours, and the survival and cell movement activity of the cells were analyzed by RTCA (xCELLigence, ACEA Biosciences, Inc.). RTCA measures the resistance value called impedance that occurs during cell growth, and various cell responses (changes in cell number, proliferation rate, cell size, cell shape, and substrate attachment quality) can be automatically measured in a kinetic manner. The resistance value detected by the cell is expressed as an indicator called Cell Index (CI), and changes in the CI value indicate changes in cell shape.

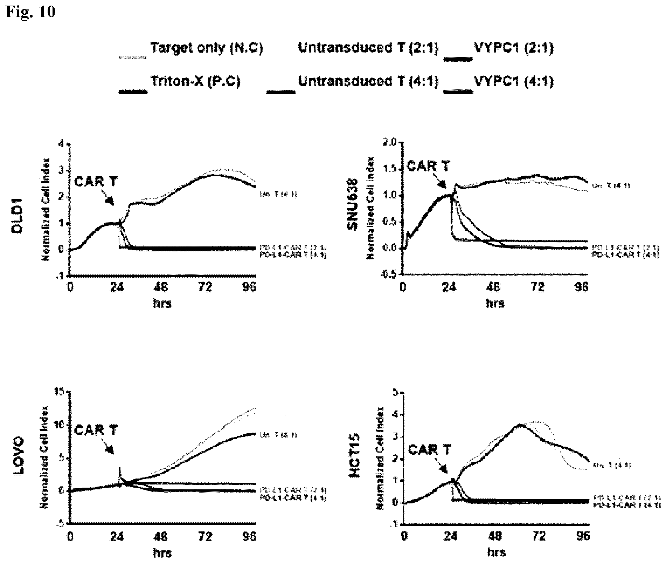

As shown in Figure 10, as a result of comparing the killing ability of transduced anti-PD-L1 CAR T cells (VYPC1) and untransduced human T cells (control group) against cancer cells, it was confirmed that when treated with VYPC1 of the present invention, the cancer cells were completely killed in both the large intestine cancer cell lines DLD1, LOVO and HCT15; and the gastric cancer cell line SNU638.

Therefore, it can be seen that the anti-PD-L1 CAR T cells (VYPC1) according to the present invention have excellent cancer cell killing ability against malignant tumor cells including gastric cancer and large intestine cancer expressing PD-L1.

### [Example 10]

### Evaluation of cancer cell killing ability of immune cells (VYPC1 and VYPC2) expressing PD-L1 specific chimeric antigen receptor against gastric cancer and glioblastoma

In order to evaluate the anticancer ability of the anti-PD-L1 CAR T cells (VYPC1) produced in Example 3 above against the PD-L1 specific antigen, the gastric cancer cell line SNU638, the glioblastoma cell line U251, and the large intestine cancer cell line DLD1 were each co-cultured with the anti-PD-L1 CAR T cells (VYPC1 and VYPC2) to measure the cancer cell killing ability.

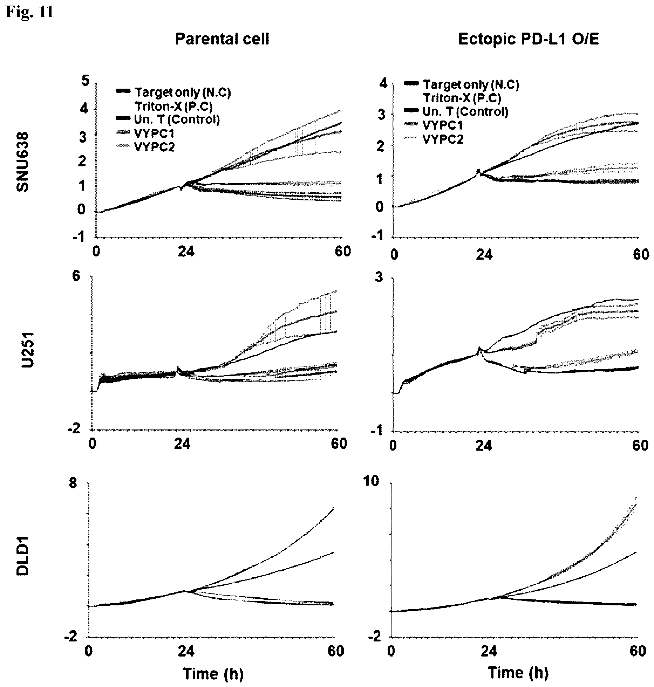

As a result, as shown in Figure 11, as a result of comparing the killing ability of transduced anti-PD-L1 CAR T cells (VYPC1 and VYPC2) and untransduced human T cells (control group) against cancer cells, it was confirmed that when treated with VYPC1 or VYPC2 of the present invention, the cancer cells were killed in parental cells or the gastric cancer cell line SNU638; the glioblastoma cell line U251; the large intestine cancer cell line DLD1 in which PD-L1 is overexpressed (PD-L1 O/E).

Therefore, it can be seen that the anti-PD-L1 CAR T cells (VYPC1 and VYPC2) according to the present invention have excellent cancer cell killing ability against gastric cancer, large intestine cancer, and glioblastoma.

### [Example 11]

### Evaluation of in vitro cell killing ability immune cells (VYPC1) expressing PD-L1 specific chimeric antigen receptor against normal cells

In order to evaluate the anticancer ability of the anti-PD-L1 CAR T cells (VYPC1) produced in Example 3 above against the PD-L1 specific antigen, the normal kidney cell line HEK 293T was co-cultured with the anti-PD-L1 CAR T cells (VYPC1) to measure the cell killing ability.

In the same manner as in Example 8 above, the gastric cancer cell line SNU638, the normal kidney cell line HEK 293T (transformed with SV40 Large T) that does not express PD-L1, and the kidney cell line HEK 293T PDL1 O/E that overexpresses the PD-L1 antigen were co-cultured with the anti-PD-L1 CAR T cells (VYPC1). At this time, the culture and co-culture of VYPC1 were each divided into cytokines IL-2 and IL-7+IL-15.

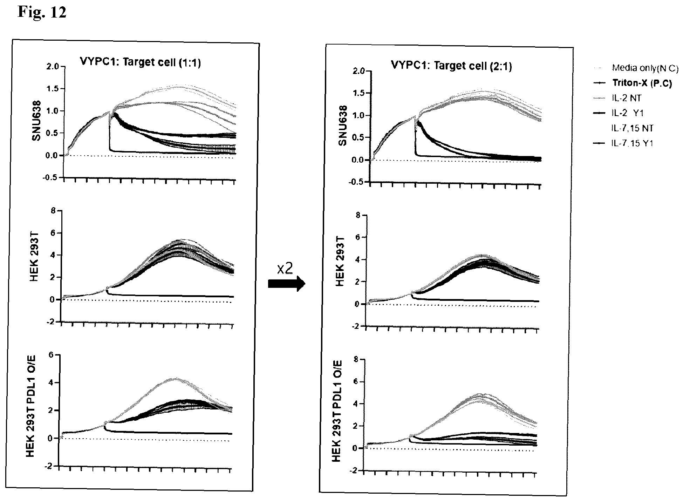

As a result, as shown in Figure 12, it was shown that in the case of the gastric cancer cell line (SNU638), the cancer cells were killed, and in particular, it was shown that when co-cultured with twice the amount of VYPC1 compared to the cancer cell line, the cancer cells were completely killed. In addition, it was shown that the normal cell line (HEK293 T) that does not express PD-L1 are not affected by cell death by VYPC1, and in the case of HEK 293T (HEK 293T PDL1 O/E) that overexpresses PD-L1, cells can be completely killed by a relatively large amount of VYPC1, unlike the killing effect shown in cancer cells.

Therefore, it was confirmed that the anti-PD-L1 CAR T cells (VYPC1) according to the present invention have no killing ability at all against normal cells that do not express PD-L1, and have a low killing ability against normal cells that express PD-L1 compared to the clear killing ability against cancer cells, and thus have weak side effects against on-target and off-tumor and have safety.

### [Example 12]

### Evaluation of antitumor ability of immune cells (VYPC1) expressing PD-L1 specific chimeric antigen receptor in liver cancer mouse model

All animal experiments were conducted with the approval from the Institutional Animal Care and Use Committee (IACUC, Chonnam National University, Republic of Korea). Four-week-old female NSG (NOD SCID gamma) mice were supplied from the SPF Animal Laboratory of Vaccine Center at Hwasun, and the human-derived hepatocellular carcinoma cell lines HepG2 and Hep3B were supplied from the Korean Cell Line Bank.

A human tumor xenograft mouse model was produced by subcutaneously injecting 100 ul of a solution containing the human-derived hepatocellular carcinoma cell lines HepG2 (2.5 × 10⁶ cells) and Hep3B (2.5 × 10⁶ cells) each mixed with high-concentration Matrigel (Corning) at a ratio of 1:1 into the right flank of the mouse. When the tumor size reached 100 to 200 mm³, each group was intravenously injected once with PBS, the control T cells (activated control normal T cells not transduced with a virus; Cont-T), and the anti-PD-L1 CAR T cells (VYPC1) at an amount of 1 × 10⁷/200 ul. Four to five mice were used per group, and the tumor size and body weight of the mice were measured twice a week. The body weight of the mice can be used to determine whether the body condition of the mice has recovered and whether there are any side effects due to administration.

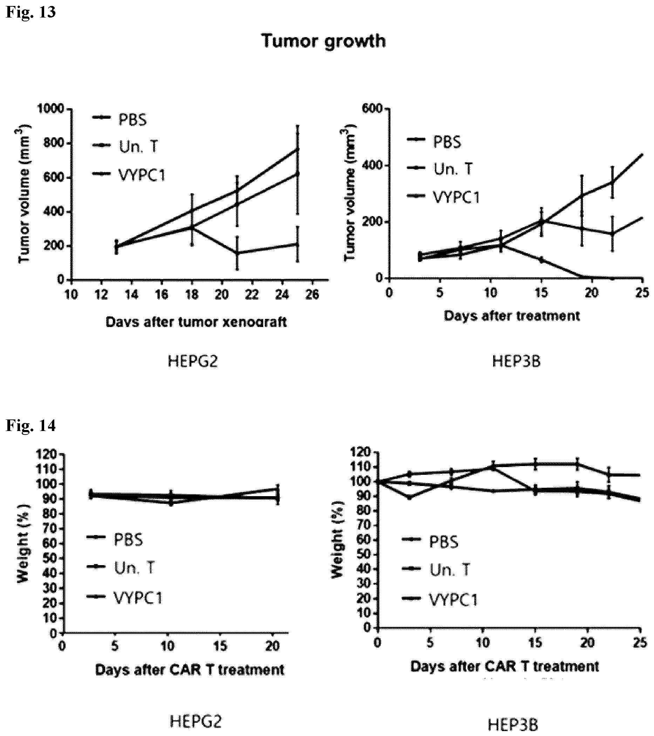

As shown in Figure 13, as a result of measuring the tumor size, it was confirmed that the tumor size of the mouse group administered the anti-PD-L1 CAR T cells (VYPC1) of the present invention was significantly reduced after 18 days in the case of HEPG2 and after 15 days in the case of HEP3B compared to that of the control group.

In addition, as shown in Figure 14, as a result of measuring the body weight of the mouse, it was shown that the body weight loss was recoverd after 10 days of administration in the mouse group administered the anti-PD-L1 CAR T cells (VYPC1) of the present invention, and through this, it was confirmed that the health condition was quickly recovered.

Therefore, it can be seen that the anti-PD-L1 CAR T cells (VYPC1) of the present invention have an excellent anticancer effect.

### [Example 13]

### Evaluation of antitumor ability of immune cells (VYPC1) expressing PD-L1 specific chimeric antigen receptor in gastric cancer mouse model

All animal experiments were conducted with the approval from the Institutional Animal Care and Use Committee (IACUC, Chonnam National University, Republic of Korea). Four-week-old female NSG (NOD SCID gamma) mice were supplied from the SPF Animal Laboratory of Vaccine Center at Hwasun, and the human-derived gastric cancer cell line SNU638 was supplied from the Korean Cell Line Bank.

A human tumor xenograft mouse model was produced by subcutaneously injecting 100 ul of a solution containing the human-derived gastric cancer cell line SNU638 (2 × 10⁶ cells) mixed with high-concentration Matrigel (Corning) at a ratio of 1:1 into the right flank of the mouse. When the tumor size reached 50 mm³, each group was intravenously injected once with PBS, the high-dose control T cells (activated control normal T cells not transduced with a virus, 10 × 10⁶ cells /200 ul; Cont-T), and the anti-PD-L1 CAR T cells (VYPC1) divided into three groups: a low dose (1 × 10⁶ cells/200 ul), a medium dose (5 × 10⁶ cells/200 ul), and a high dose (10 × 10⁶ cells/200 ul). Seven mice were used per group, and the tumor size and body weight of the mice were measured twice a week. The body weight of the mice can be used to determine whether the body condition of the mice has recovered and whether there are any side effects due to administration.

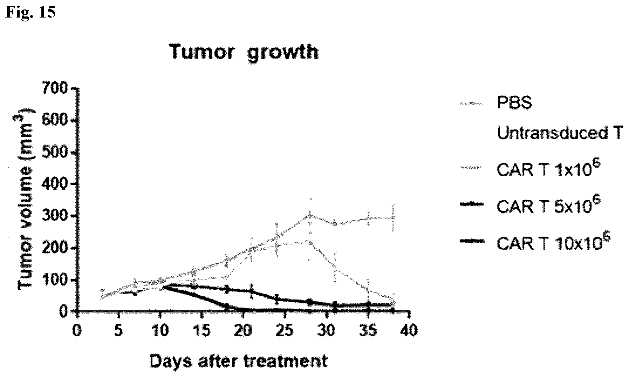

As shown in Figure 15, as a result of measuring the tumor size, it was confirmed that the tumor size of the mouse group administered the anti-PD-L1 CAR T cells (VYPC1) of the present invention was significantly reduced after 13 days compared to that of the control group. In particular, the anti-PD-L1 CAR T cells (VYPC1) of the present invention showed a more excellent effect when administered at a high dose.

Therefore, it can be seen that the anti-PD-L1 CAR T cells (VYPC1) of the present invention have an excellent anticancer effect even in experiments using small animals.

## Claims

1. A chimeric antigen receptor (CAR) comprising a binding domain that specifically binds to PD-L1 (programmed death-ligand 1).

2. The chimeric antigen receptor according to claim 1, wherein the chimeric antigen receptor further comprises at least one selected from the group consisting of a signal sequence, a hinge and transmembrane domain (TM), and an intracellular domain.

3. The chimeric antigen receptor according to claim 1, wherein the binding domain that specifically binds to PD-L1 is a single chain variable fragment (scFv) of an anti-PD-L1 antibody.

4. The chimeric antigen receptor according to claim 3, wherein the single chain variable fragment (scFv) of an anti-PD-L1 antibody comprises the amino acid sequence of SEQ ID NO: 8.

5. The chimeric antigen receptor according to claim 2, wherein the signal sequence comprises a signal sequence of CD8α.

6. The chimeric antigen receptor according to claim 5, wherein the signal sequence of CD8α comprises the amino acid sequence of SEQ ID NO: 7.

7. The chimeric antigen receptor according to claim 2, wherein the hinge and transmembrane domain comprises a hinge and transmembrane domain of CD8α.

8. The chimeric antigen receptor according to claim 7, wherein the hinge and transmembrane domain of CD8α comprises the amino acid sequence of SEQ ID NO: 9.

9. The chimeric antigen receptor according to claim 2, wherein the intracellular domain comprises an intracellular signal region sequence selected from the group consisting of CD28, 4-1BB and CD3ζ, or a sequence selected from the group consisting of a combination thereof.

10. The chimeric antigen receptor according to claim 9, wherein the intracellular signal region sequence of CD28 comprises the amino acid sequence of SEQ ID NO: 10, the intracellular signal region sequence of 4-1BB comprises the amino acid sequence of SEQ ID NO: 11, and the intracellular signal region sequence of CD3ζ comprises the amino acid sequence of SEQ ID NO: 12.

11. A polynucleotide comprising a nucleic acid sequence encoding the chimeric antigen receptor according to any one of claims 1 to 10.

12. An expression vector comprising the polynucleotide according to claim 11.

13. A virus comprising the expression vector according to claim 12.

14. An immune cell expressing the chimeric antigen receptor according to any one of claims 1 to 10 on the surface thereof.

15. The immune cell according to claim 14, wherein the immune cell is a T cell, a MIL cell, a TIL cell, an NK cell, an NKT cell, a DC cell, and a cell derived from iPSC.

16. A pharmaceutical composition for preventing or treating cancer, comprising the immune cell according to claim 14.
